# EUROPEAN PATENT SPECIFICATION

(11) **EP 2 170 284 B1**
(45) Date of publication and mention of the grant of the patent: **24.08.2011**
(21) Application number: 08779037.4
(22) Date of filing: 17.07.2008
(51) Int. Cl.: A61K 9/14, B01J 3/00, B01D 9/00

(54) **A METHOD OF PREPARING A PHARMACEUTICAL CO-CRYSTAL COMPOSITION**
VERFAHREN ZUR HERSTELLUNG EINER PHARMAZEUTISCHEN CO-KRISTALL-ZUSAMMENSETZUNG
PROCÉDÉ DE PRÉPARATION D'UNE COMPOSITION PHARMACEUTIQUE À CO-CRYSTAL

(30) Priority: 18.07.2007 EP 07112692
(43) Date of publication of application: 07.04.2010
(73) Proprietor: Feyecon B.V., 2012 LG Haarlem (NL)
(72) Inventor: VAN ROSMALEN, Gerda Maria, NL-2625 LL Delft (NL); HOFLAND, Gerard Willem, NL-1382 GS Weesp (NL)
(74) Representative: Swinkels, Bart Willem
(86) International application number: PCT/NL2008/050490
(87) International publication number: WO 2009/011584

(56) References cited:
- WO-A-01/66091
- WO-A-2007/053536
- US-A- 5 424 076
- ADIVARAHA JAYASANKAR ET AL: "Cocrystal Formation during Cogrinding and Storage is Mediated by Amorphous Phase" PHARMACEUTICAL RESEARCH, KLUWER ACADEMIC PUBLISHERS-PLENUM PUBLISHERS, NE, vol. 23, no. 10, 19 September 2006 (2006-09-19), pages 2381-2392, XP019437062 ISSN: 1573-904X
- MONEGHINI M ET AL: "Characterisation of nimesulide-betacyclodextrins systems prepared by supercritical fluid impregnation" EUROPEAN JOURNAL OF PHARMACEUTICS AND BIOPHARMACEUTICS, ELSEVIER SCIENCE PUBLISHERS B.V., AMSTERDAM, NL, vol. 58, no. 3, 1 November 2004 (2004-11-01), pages 637-644, XP004586445 ISSN: 0939-6411

## Description

### TECHNICAL FIELD OF THE INVENTION

The present invention relates to a method of preparing a pharmaceutical co-crystal composition. More particularly, the present invention aims to provide a method of preparing a pharmaceutical co-crystal composition that contains virtually no solvent residue.

### BACKGROUND OF THE INVENTION

Undesirable physicochemical properties, physiological barriers, or issues of toxicity often limit the therapeutic benefit of pharmaceutically active ingredients. This has motivated research in drug delivery systems for poorly soluble, poorly absorbed and labile substances. Crystalline self-assemblies of pharmaceutically active substances represent a promising delivery modality for improving drug solubility, dissolution rate, stability and bioavailability.

These crystalline systems offer various advantages over e.g. amorphous delivery systems both from design and stability perspectives. However, the existence of more than one crystalline form of a given compound, typically in the form of polymorphs, represents both a problem and an opportunity.

Crystalline polymorphs typically have different solubilities from one another, such that a more thermodynamically stable polymorph is less soluble than a less thermodynamically stable polymorph. Pharmaceutical polymorphs can also differ in properties such as shelf-life, bioavailability, morphology, vapour pressure, density, colour, and compressibility. Accordingly, variation of the crystalline state of a pharmaceutically active component is one of many ways in which to modulate the physical properties thereof. However, for most pharmaceutically active substance no polymorphs are available that exhibit the desired combination of pharmacokinetic and pharmacodynamic properties. An important problem observed with many polymorphs is their inherent thermodynamic instability.

In order to realise the full benefits associated with crystalline forms of pharmaceutically active substances without incurring significant drawbacks such as instability and low solubility, it has been proposed to prepare co-crystals of these pharmaceutically active substances and a suitable co-builder.

WO 2006/007448, for instance, describes a pharmaceutical co-crystal composition comprising an active pharmaceutical ingredient (API) and a co-builder, such that the API and co-builder are capable of co-crystallising from a solid or solution phase under crystallisation conditions. According to WO 2006/007448 these pharmaceutical co-crystal compositions have improved properties, in particular, as oral formulations. More particularly, it is mentioned therein that co-crystals offer the possibility to increase or decrease the dissolution rate of API-containing pharmaceutical compositions in water, increase or decrease the bioavailability of orally-administered compositions, and provide a more rapid or more delayed onset to therapeutic effect.

The examples of WO 2006/007448 describe a process in which the API and the co-builder are dissolved in an organic solvent, if needed by heating the mixture to a sufficiently high temperature to obtain a homogeneous solution. Next, the solvent is slowly evaporated, optionally after having cooled down the homogeneous solution to room temperature. An important drawback of this process resides in the fact that it is difficult to completely remove the solvent, especially if the API or co-builder is very labile.

WO 2007/053536 describes a method for making pharmaceutical co-crystals by precipitation of a solid form from a solvent, wherein the pharmaceutical co-crystal is a solid molecular complex between two or more reactants where the reactants are held in the complex by non-ionic interactions, and wherein one of the reactants is an active pharmaceutical ingredient, the method comprising combining the active pharmaceutical ingredient, other reactant, and solvent under over saturation conditions with respect to the complex in the solution, wherein one of the active pharmaceutical ingredient, and the other reactant or reactants is provided in a molar excess of at least 2:1 with respect to its presence in the complex. A drawback of the method described in the international patent application resides in the fact that organic solvents are required for the preparation of certain co-crystals. Furthermore, it is difficult to completely remove the solvent, especially if the pharmaceutical ingredient or reactant is very labile.

Consequently, there is a need for a method that enables easy preparation of a pharmaceutical co-crystal composition that contains virtually no solvent residue and a method that can suitably be used to prepare co-crystals of highly labile pharmaceutically active ingredients.

### SUMMARY OF THE INVENTION

The inventors have developed a method that meets the aforementioned requirements. The method according to the present invention uses a supercritical or liquefied gas to prepare a co-crystallisation medium containing a dissolved pharmaceutically active component and a dissolved co-builder by simultaneously contacting the supercritical or liquefied gas with solid particles of the pharmaceutically active component and solid particles of the co-builder. The solid particles of the pharmaceutically active component and the solid particles of the co-builder are subsequently converted into co-crystals within the co-crystallisation medium by keeping the gas in a supercritical or liquid state until the bulk of the pharmaceutically active component has been incorporated in the crystal matrix of said co-crystals. Once the co-crystals have formed, these crystals can simply be recovered by separating these co-crystals from the supercritical or liquefied gas by depressurisation.

It is an essential element of the present method that at least a fraction of the pharmaceutically active component and at least a fraction of the co-builder remain in an undissolved state during the co-crystallisation process. Thus, the present method is ideally suited for preparing co-crystals from a wide variety of staring materials, including those that have limited solubility in the supercritical or liquefied gas. The present method does not require the use of liquid solvents and thus enables production of solvent-free co-crystals under very mild conditions.

### DETAILED DESCRIPTION OF THE INVENTION

Accordingly, the present invention relates to a method of preparing a pharmaceutical co-crystal composition, said method comprising the steps of:
a. simultaneously contacting a supercritical or liquefied gas with solid particles of a pharmaceutically active component and with solid particles of a co-builder to form a co-crystallisation medium containing dissolved pharmaceutically active component and dissolved co-builder as well as solid particles of the pharmaceutically active component and solid particles of the co-builder;
b. transforming the solid particles of the pharmaceutically active component and the solid particles of the co-builder into co-crystals of said pharmaceutically active component and said co-builder by keeping the supercritical or liquefied gas in a supercritical or liquid state until at leat 80 wt.%, preferably at least 90 wt.% of the pharmaceutically active component is incorporated in the crystal matrix of said co-crystals; and
c. separating said co-crystals from the supercritical or liquefied gas; wherein at least a fraction of the pharmaceutically active component and at least a fraction of the co-builder remain in an undissolved state during the co-crystallisation.

The term "co-crystal" as referred to herein refers to a crystalline material comprised of two or more unique solids at room temperature (20 °C), each containing distinctive physical characteristics, such as structure, melting point and heats of fusion. The co-crystals of the present invention comprise a co-builder bonded to a pharmaceutically active component.

The term "pharmaceutical co-crystal" as used herein describes a co-crystal wherein at least one of the components is a pharmaceutically active component.

The term "co-builder" refers to a material with a melting point above room temperature that is capable of forming a co-crystal as defined herein.

The present method offers the advantage that neither the pharmaceutically active component nor the co-builder need to be pre-dissolved before introduction into to the co-crystallisation medium. Hence, in accordance with a particularly preferred embodiment of the invention, both the pharmaceutically active component and the co-builder are in an undissolved when combined with the supercritical or liquefied gas. Even more preferably, both the pharmaceutically active component and the co-builder are in a solid state when combined with the supercritical or liquefied gas in step a.

The formation of co-crystals in the present method may occur through different mechanisms that can happen concurrently. According to one mechanism nuclei of co-crystals are formed in the co-crystallisation medium and grow to become co-crystal particles. According to another mechanism co-crystals are formed on the surface of particles of the pharmaceutically active component and/or on the surface of particles of the co-builder, following which these particles are gradually transformed into co-crystal particles. Although the inventors do not wish to be bound by theory, it is believed that the latter mechanism may explain the relatively high rate at which co-crystals can be formed in the present method even if the starting materials employed have an extremely low solubility in the supercritical or liquefied gas.

In accordance with a particularly preferred embodiment, the co-crystallisation medium used in the present method is saturated with both the pharmaceutically active component and the co-builder.

The method of the present invention may be operated in different ways. The solid particles of the pharmaceutically active component and the co-builder may be present in the co-crystallisation medium in the form of an intimate mixture. However, it is also feasible, to hold the solid particles of the pharmaceutically active component and the solid particles of the co-binder in separate chambers whilst ensuring that both solid materials remain in contact with the liquid or supercritical part of the co-crystallisation medium. The latter option offers the advantage that the conditions in each chamber can be controlled independently, especially if the liquid or supercritical part of the co-crystallisation medium is recirculated through both chambers.

The inventors have found that in the present method co-crystallisation can be accelerated by providing conditions that increase the number of collisions between the solid particles of the pharmaceutically active ingredient and the solid particles of the co-builder. Thus, in accordance with a preferred embodiment, the co-crystallisation medium is subjected to mixing. According to a particularly preferred embodiment, mixing is achieved by moving a plurality of solid inert objects, e.g. metal balls, through the co-crystallisation medium and allowing these objects to collide with one another. Typically, the impact energy applied in this type of mixing exceeds 30 J/m².

The supercritical or liquefied gas may suitably contain a co-solvent, notably a low boiling co-solvent. However, the method of the present method preferably does not utilise significant quantities of high boiling solvents as these may contaminate the final co-crystal product. Accordingly, in a preferred embodiment, throughout step a., even more preferably throughout steps a. and b., the supercritical or liquefied gas contains less than 10 wt.% of solvents with a boiling point of more than 80 °C. Even more preferably, the supercritical or liquefied gas contains less than 5 wt.%, most preferably less than 3 wt.% of solvents with a boiling point of more than 80 °C.

According to a particularly preferred embodiment, the solids in the co-crystallisation medium are subjected to stirring, recirculation, gas injection, boiling, shaking, other means of mechanical action or combinations thereof. Thus, it can be ensured that the co-crystallisation process proceeds as fast as is possible under the conditions employed during co-crystallisation.

An easy way to ensure that co-crystallisation occurs at the highest possible rate is to keep both the solid particles of the pharmaceutically active component and the solid particles of the co-builder in suspension during step b.

As explained herein before, it is an essential aspect of the invention that at least a fraction of the pharmaceutically active component and at least a fraction of the co-builder remain in an undissolved state during the co-crystallisation process. According to a particularly preferred embodiment, not more than 5% of the pharmaceutically active component is in a dissolved state during steps a. and b. The amount of pharmaceutically active component that is in a dissolved state during step a. and b. typically is less than 10 mg/l, preferably said amount is in the range of 0.1-2 mg/l.

Advantageously not more than 10% of the co-builder is in a dissolved state during steps a. and b. The amount of co-builder in a dissolved state typically is less than 10 mg/l, preferably 0.1-5 mg/l.

The supercritical gas or liquefied gas employed in the present process is preferably selected from the group consisting of carbon dioxide, nitrous oxide, ethane, ethylene propane, cyclopropane, propylene, butane, argon, nitrogen and mixtures thereof. According to a particularly preferred embodiment, the supercritical gas or liquefied gas contains at least 50 wt.%, more preferably at least 80 wt.% of carbon dioxide. Most preferably the supercritical gas or liquefied gas employed in the present method is supercritical or liquefied carbon dioxide.

The liquefied gas or supercritical gas employed in the present method has a pressure of at least 5 bar, preferably of at least 30 bar. According to a particularly preferred embodiment, the supercritical or liquefied gas has a pressure within the range of 70-500 bar. The temperature of the supercritical or liquefied gas is preferably in the range of 0-100 °C, more preferably in the range of 30-100 °C.

Particularly good results can be obtained with the present method employs a supercritical co-crystallisation medium. Hence, in a preferred embodiment, both steps a. and b. are executed under supercritical conditions.

The inventors have found that the present method can deliver high yields of co-crystals at a relatively high production rate, if the solid particles of the pharmaceutically active component employed in step a., have a very small particle size, e.g. a mass weighted average diameter of less than 50 µm. Likewise, it is also preferred to employ the co-builder in the form of a powder with a very small particle size. Accordingly, in another preferred embodiment, the solid particles of the co-builder that are employed in step a. have a mass weighted average diameter of less than 50 µm. In order to ensure that the particles of the pharmaceutically active component and the particles of the co-builder contained in the co-crystallisation medium have such a small particle size, it is preferred to add said components in the form of a powder having a mass weighted average diameter of less than 50 µm.

The present method can produce high yields of non-contaminated co-crystals in less than 12 hours or even in less than 8 hours. Hence, according to a preferred embodiment, the combined duration of steps a. and b. does not exceed 8 hours.

The benefits of the present method are particularly pronounced when the method is used to prepare co-crystals of pharmaceutically active components that exhibit poor water solubility. The present method enables the production of co-crystals of poorly water-soluble pharmaceutically active components that have considerably higher oral bioavailabilty than crystals made of the pure component. According to a particularly preferred embodiment of the present method, the pharmaceutically active component employed in step a. has a water solubility at 37 °C of less than 5 mg/l. The co-builder employed in step a. preferably has a water solubility at 37 °C of 5-50 mg/l.

In the present method the co-crystals obtained typically contain 10-90 wt.% of the pharmaceutically active component and 90-10 wt.% of the co-builder. More preferably, the co-crystals contain 20-80 wt.% of the pharmaceutically active component and 80-20 wt.% of the co-builder.

Together, the pharmaceutically active component and the co-builder typically represent at least 90 wt.% of the co-crystals obtained in the present method. Most preferably, the combination of pharmaceutically active component and co-builder represent at least 95 wt.%, most preferably at least 98 wt.% of the co-crystals formed.

The present method is particularly suitable for the preparation of co-crystals of the following compound combinations: (Lewis) acid-base combinations often involving H-bonding, such as carboxylic acid-amines, alcohols-ketones, aldehydes-ketones; combinations exhibiting pi-stacking between adjacent molecules within the crystal matrix (e.g., small HOMO/LUMO gaps), such as double bond/conjugate double bond combinations; combinations of compounds comprising aromatic rings and compounds comprising cationic groups.

According to a particularly preferred embodiment, the pharmaceutically active component comprises at least one functional group selected from ether, thioether, alcohol, thiol, aldehyde, ketone, thioketone, nitrate ester, phosphate ester, thiophosphate ester, ester, thioester, sulfate ester, carboxylic acid, phosphinic acid, phosphonic acid, sulfonic acid, amide, primary amine, secondary amine, ammonia, tertiary amine, imine, thiocyanate, cyanamide, oxime, nitrile diazo, organohalide, nitro, S-heterocyclic ring (e.g. thiophene), N-heterocyclic ring (e.g. pyrrole, pyridine), O-heterocyclic ring (e.g furan), epoxide, peroxide, hydroxamic acid, imidazole.

The co-builder advantageously comprises at least one functional group selected from amine, amide, pyridine, imidazole, indole, pyrrolidine, carbonyl, carboxyl, hydroxyl, phenol, sulfone, sulfonyl, mercapto and methyl thio.

Examples of pharmaceutically active components that may suitably be incorporated in co-crystals using the present method include carbamazepine, norfloxacin, naphthoic acid derivatives, fluorouracil derivatives, azidothymidine, fluoxetine, caffeine, olanzapine and combinations thereof.

Examples of co-builders that can advantageously be employed in the present method include amides such as nicotinamide and benzamide, saccharin, amino acids, benzoic acids and combinations thereof.

The size of the co-crystals formed in the present method can vary considerably, depending on the conditions employed during co-crystallisation. Advantageously, the co-crystals obtained from step c. of the present method have a mass weighted average diameter of 5-100 µm.

The invention is further illustrated by means of the following examples.

### EXAMPLES

The following examples describe the preparation of pharmaceutical co-crystals in accordance with the present invention. Both the pharmaceutically active component and the co-builder were gently ground to a fine powder. The resulting powders were transferred into a high pressure vessel (250 ml) together with 20 stainless steel balls (5 mm) to enhance impact of contacting and mixing of the powder. Subsequently, the vessel was immersed in a temperature controlled water bath and pressurised with carbon dioxide. During the co-crystallisation process the vessel was vertically rotated at a rotation speed of 30 rpm. At the end of the experiment the vessel was depressurised and the remaining contents of the vessel were removed for analysis by means of Raman spectroscopy.

### Example 1

Carbamazepine (1 g) and nicotinamide (1 g) were mixed in the high pressure vessel in presence of 155 g of CO₂ (40°C, 100 bar) for 1.5 hours.

Raman analysis showed that photon count maxima at Raman shifts typical for crystalline carbamazepine had completely vanished, count maxima for crystalline nicotinamide had decreased dramatically and new photon count maxima not representing carbamazepine or nicotinamide appeared, indicating complete conversion of the carbamazepine.

### Example 2

Carbamazepine (1 g) and saccharin (1 g) were mixed in the high pressure vessel in the presence of 155 g of CO₂ (40°C, 100 bar) for 1.5 hours.

Similar to example 1, Raman analysis indicated complete conversion of carbamazepine.

### Example 3

Carbamazepine (1 g) and nicotinamide (1 g) were transferred into the high pressure vessel in presence of CO₂. The resulting co-crystallisation medium was kept at 40°C and 100 bar for 1.5 hours. However, this time the high pressure vessel was not rotated.

Raman analysis indicated that the starting materials had only partially been converted to co-crystals.

### Example 4

Example 2 was repeated except that the co-crystallisation process was discontinued after 40 minutes by depressurisation.

Raman analysis indicated partial conversion.

## Claims

1. A method of preparing a pharmaceutical co-crystal composition, said method comprising the steps of:
a. simultaneously contacting a supercritical or liquefied gas with solid particles of a pharmaceutically active component and with solid particles of a co-builder to form a co-crystallisation medium containing the pharmaceutically active component and the co-builder as well as solid particles of the pharmaceutically active component and solid particles of the co-builder;
b. transforming the solid particles of the pharmaceutically active component and the solid particles of the co-builder into co-crystals of said pharmaceutically active component and said co-builder by keeping the supercritical or liquefied gas in a supercritical or liquid state until at least 80 wt.%, preferably at least 90 wt.% of the pharmaceutically active component is incorporated in the crystal matrix of said co-crystals; and
c. separating said co-crystals from the supercritical or liquefied gas;
wherein at least a fraction of the pharmaceutically active component and at least a fraction of the co-builder remain in an undissolved state during the co-crystallisation.

2. Method according to claim 1, wherein the co-crystallisation medium is saturated with both the pharmaceutically active component and the co-builder.

3. Method according to any one of the preceding claims, wherein the solids in the co-crystallisation medium are subjected to stirring, recirculation, gas injection, boiling, shaking, other means of mechanical action or combinations thereof.

4. Method according to any one of the preceding claims, wherein not more than 5% of the pharmaceutically active component is in a dissolved state during steps a. and b.

5. Method according to any one of the preceding claims, wherein not more than 10% of the co-builder is in a dissolved state during step a. and b.

6. Method according to any one of the preceding claims, wherein the supercritical or liquefied gas contains a gas selected from the group consisting of carbon dioxide, nitrous oxide, ethane, ethylene propane, cyclopropane, propylene, butane, argon, nitrogen and mixtures thereof.

7. Method according to any one of the preceding claims, wherein the supercritical or liquefied gas has a pressure within the range of 70-500 bar.

8. Method according to any one of the preceding claims, wherein the supercritical or liquefied gas has a temperature in the range of 30-100 °C.

9. Method according to any one of the preceding claims, wherein the solid particles of the pharmaceutically active component that are employed in step a. have a mass weighted average diameter of less than 50 µm.

10. Method according to any one of the preceding claims, wherein the solid particles of the co-builder that are employed in step a. have a mass weighted average diameter of less than 50 µm.

11. Method according to any one of the preceding claims, wherein the pharmaceutically active component employed in step a. has a water solubility at 37 °C of less than 5 mg/l.

12. Method according to any one of the preceding claims, wherein the co-crystals contain 20-80 wt.% of the pharmaceutically active component and 80-20 wt.% of the co-builder.

## Patentansprüche

1. Verfahren zum Herstellen einer pharmazeutischen Co-Kristall-Zusammensetzung, wobei das Verfahren die Schritte umfasst:
a. gleichzeitiges Kontaktieren eines superkritischen oder verflüssigten Gases mit Festkörperpartikeln einer pharmazeutisch aktiven Komponente und mit Festkörperpartikeln eines Cobuilders um ein Co-Kristallisations-Medium zu bilden, das die pharmazeutisch aktive Komponente und den Cobuilder sowie Festkörperpartikel der pharmazeutisch aktiven Komponente und Festkörperpartikel des Cobuilders enthält;
b. Umwandeln der Festkörperpartikel der pharmazeutisch aktiven Komponente und der Festkörperpartikel des Cobuilders in Co-Kristalle der pharmazeutisch aktiven Komponente und des Cobuilders durch Bewahren des superkritischen oder verflüssigten Gases in einem superkritischen oder verflüssigten Zustand bis wenigstens 80 Gew.-%, bevorzugt wenigstens 90 Gew.-% der pharmazeutisch aktiven Komponente in die Kristallmatrix des Co-Kristalls inkorporiert ist; und
c. Trennen der Co-Kristalle von dem superkritischen oder verflüssigten Gas;
wobei wenigstens ein Anteil der pharmazeutisch aktiven Komponente und wenigstens ein Anteil des Cobuilders während der Co-Kristallisation in einem ungelösten Zustand verbleiben.

2. Verfahren nach Anspruch 1, wobei das Co-Kristallisations-Medium mit der pharmazeutisch aktiven Komponente und dem Cobuilder gesättigt ist.

3. Verfahren nach einem beliebigen der vorhergehenden Ansprüche, wobei die Festkörper in dem Co-Kristallisations-Medium Rühren, Rezirkulation, Gasinjektion, Sieden, Schütteln, anderen Mitteln mechanischer Einwirkung oder Kombinationen davon unterzogen werden.

4. Verfahren nach einem beliebigen der vorhergehenden Ansprüche, wobei sich nicht mehr als 5% der pharmazeutisch aktiven Komponente in einem gelösten Zustand während der Schritte a. und b. befinden.

5. Verfahren nach einem beliebigen der vorhergehenden Ansprüche, wobei sich nicht mehr als 10% des Cobuilders in einem gelösten Zustand während der Schritte a. und b. befinden.

6. Verfahren nach einem beliebigen der vorhergehenden Ansprüche, wobei das superkritische oder verflüssigte Gas ein Gas ausgewählt aus der Gruppe bestehend aus Kohlendioxid, Stickstoffdioxid, Ethan, Ethylenpropan, Zyklopropan, Propylen, Butan, Argon, Stickstoff und Mischungen daraus enthält.

7. Verfahren nach einem beliebigen der vorhergehenden Ansprüche, wobei das superkritische oder verflüssigte Gas einen Druck im Bereich von 70-500 Bar hat.

8. Verfahren nach einem beliebigen der vorhergehenden Ansprüche, wobei das superkritische oder verflüssigte Gas eine Temperatur im Bereich von 30-100 °C hat.

9. Verfahren nach einem beliebigen der vorhergehenden Ansprüche, wobei die Festkörperpartikel der pharmazeutischen aktiven Komponente, die in Schritt a. eingesetzt werden, einen über die Masse gemessenen durchschnittlichen Durchmesser von weniger als 50 µm haben.

10. Verfahren nach einem beliebigen der vorhergehenden Ansprüche, wobei die Festkörperpartikel des Cobuilders, die in Schritt a. eingesetzt werden, einen über die Masse gemessenen durchschnittlichen Durchmesser von weniger als 50 µm haben.

11. Verfahren nach einem beliebigen der vorhergehenden Ansprüche, wobei die pharmazeutisch aktive Komponente, die in Schritt a. eingesetzt wird, bei 37°C eine Wasserlöslichkeit von weniger als 5 mg/l hat.

12. Verfahren nach einem beliebigen der vorhergehenden Ansprüche, wobei die Co-Kristalle 20-80 Gew.-% der pharmazeutisch aktiven Komponente und 80-20 Gew.-% des Cobuilders enthalten.

## Revendications

1. Procédé de préparation d'une composition de co-cristal pharmaceutique, ledit procédé comprenant les étapes consistant à :
a. mettre en contact simultanément un gaz surcritique ou liquéfié avec des particules solides d'un composant pharmaceutiquement actif et avec des particules solides d'un co-adjuvant pour former un milieu de co- cristallisation contenant le composant pharmaceutiquement actif et le co- adjuvant ainsi que des particules solides du composant pharmaceutiquement actif et des particules solides du co-adjuvant ;
b. transformer les particules solides du composant pharmaceutiquement actif et les particules solides du co-adjuvant en co-cristaux dudit composant pharmaceutiquement actif et dudit co-adjuvant en maintenant le gaz surcritique ou liquéfié dans un état surcritique ou liquide jusqu'à ce qu'au moins 80 % en poids, de préférence au moins 90 % en poids du composant pharmaceutiquement actif soient incorporés dans la matrice cristalline desdits co-cristaux ; et
c. séparer lesdits co-cristaux dudit gaz surcritique ou liquéfié ;
où au moins une fraction du composant pharmaceutiquement actif et au moins une fraction du co-adjuvant restent dans un état non dissous pendant la co-cristallisation.

2. Procédé selon la revendication 1, dans lequel le milieu de co-cristallisation est saturé à la fois avec le composant pharmaceutiquement actif et le co-adjuvant.

3. Procédé selon l'une quelconque des revendications précédentes, dans lequel les solides dans le milieu de co-cristallisation sont soumis à une agitation, une recirculation, une injection de gaz, une ébullition, un secouement, un autre moyen d'action mécanique ou leurs combinaisons.

4. Procédé selon l'une quelconque des revendications précédentes, dans lequel pas plus de 5 % du composant pharmaceutiquement actif sont dans un état dissous pendant les étapes a. et b.

5. Procédé selon l'une quelconque des revendications précédentes, dans lequel pas plus de 10 % du co-adjuvant sont dans un état dissous pendant les étapes a. et b.

6. Procédé selon l'une quelconque des revendications précédentes, dans lequel le gaz surcritique ou liquéfié contient un gaz choisi dans le groupe consistant en le dioxyde de carbone, l'oxyde nitreux, l'éthane, l'éthylène, le propane, le cyclopropane, le propylène, le butane, l'argon, l'azote et leurs mélanges.

7. Procédé selon l'une quelconque des revendications précédentes, dans lequel le gaz surcritique ou liquéfié a une pression dans la plage de 70 à 500 bar.

8. Procédé selon l'une quelconque des revendications précédentes, dans lequel le gaz surcritique ou liquéfié a une température dans la plage de 30 à 100 °C.

9. Procédé selon l'une quelconque des revendications précédentes, dans lequel les particules solides du composant pharmaceutiquement actif qui sont employées dans l'étape a. ont un diamètre moyen pondéré en masse inférieur à 50 µm

10. Procédé selon l'une quelconque des revendications précédentes, dans lequel les particules solides du co-adjuvant qui sont employées dans l'étape a. ont un diamètre moyen pondéré en masse inférieur à 50 µm.

11. Procédé selon l'une quelconque des revendications précédentes, dans lequel le composant pharmaceutiquement actif employé dans l'étape a. a une solubilité dans l'eau à 37 °C inférieure à 5 mg/L.

12. Procédé selon l'une quelconque des revendications précédentes, dans lequel les co-cristaux contiennent 20 à 80 % en poids du composant pharmaceutiquement actif et 80 à 20 % en poids du co-adjuvant.
